# EUROPÄISCHE PATENTSCHRIFT

(11) **EP 2 023 833 B1**
(45) Veröffentlichungstag und Bekanntmachung des Hinweises auf die Patenterteilung: **17.02.2010**
(21) Anmeldenummer: 07729631.7
(22) Anmeldetag: 29.05.2007
(51) Int. Cl.: A61B 17/68, A61B 17/72, A61B 17/80, A61B 17/86

(54) **IMPLANTAT MIT FIXIERELEMENT**
IMPLANT WITH A FIXING ELEMENT
IMPLANT AVEC ÉLÉMENT FIXATEUR

(30) Priorität: 31.05.2006 DE 102006026590
(43) Veröffentlichungstag der Anmeldung: 18.02.2009
(73) Patentinhaber: Aesculap AG, 78532 Tuttlingen (DE)
(72) Erfinder: GRAF, Sabine, 78532 Tuttlingen (DE); SAUERESSIG, Thomas, 78532 Tuttlingen (DE)
(74) Vertreter: Hoeger, Stellrecht & Partner Patentanwälte
(86) Internationale Anmeldenummer: PCT/EP2007/055210
(87) Internationale Veröffentlichungsnummer: WO 2007/138062

(56) Entgegenhaltungen:
- DE-A1- 4 409 833
- DE-A1- 10 320 855
- US-A1- 2001 041 894
- US-A1- 2004 034 354
- US-A1- 2005 096 657
- US-B1- 6 755 833

## Beschreibung

Die vorliegende Erfindung betrifft ein Implantat mit mindestens einer eine Durchgangsrichtung definierenden Durchbrechung zum Aufnehmen eines Befestigungselements zum Festlegen des Implantats an oder in einem menschlichen oder tierischen Körper, wobei das Implantat mindestens ein Fixierelement umfasst, welches derart am Implantat angeordnet ist und mindestens teilweise in die mindestens eine Durchbrechung vorsteht, dass eine freie Querschnittsfläche der mindestens einen Durchbrechung durch das mindestens eine Fixierelement mindestens teilweise verkleinert wird, wobei das Fixierelement, ohne die mindestens eine Durchbrechung vollständig zu umgeben, am Implantat angeordnet ist.

Implantate der eingangs beschriebenen Art dienen häufig zur Verbindung zweier Knochenteile und werden mit Befestigungsmitteln, zum Beispiel Knochenschrauben oder Nägeln, an den Knochenteilen festgelegt. Beispielsweise durchsetzt dann eine Knochenschraube die Durchbrechung des Implantats und wird mit ihrem Schraubenkörper in eines der Knochenteile eingeschraubt. Es ist insbesondere aus der DE 203 07 265 U1 bekannt, die Durchbrechung zum Aufnehmen eines Befestigungselements im Querschnitt mittels eines Fixierkörpers zu verkleinern. Dieser Fixierkörper dient dazu, eine Relativbewegung zwischen der Knochenschraube und dem Implantat abzubremsen, um das Implantat möglichst spielfrei an den Knochenteilen festzulegen. Ferner wird so auch das Ausdrehen der Knochenschraube bei Körperbewegungen unterbunden. Nachteilig bei dem aus der DE 203 07 265 U1 bekannten Implantat ist jedoch dessen relativ aufwändige Herstellung. So muss der Fixierkörper mit einer Öffnung versehen werden, die zudem fluchtend mit einer eine Durchbrechung bildenden Gewindebohrung der Knochenschraube auszurichten ist.

Aus der US 2004/0034354 A1 sowie der US 6,755,833 B1 sind Knochenhaltevorrichtungen bekannt.

Es ist daher Aufgabe der vorliegenden Erfindung, ein Implantat der eingangs beschriebenen Art so zu verbessern, dass es bei gleicher Funktionalität einfacher herzustellen ist.

Diese Aufgabe wird bei einem Implantat der eingangs beschriebenen Art erfindungsgemäß dadurch gelöst, dass das mindestens eine Fixierelement an der Durchbrechung und/oder in einer Fixierelementaufnahme unbeweglich festgelegt ist.

Die erfindungsgemäße Weiterbildung hat den Vorteil, dass das Fixierelement selbst nicht mit einer Öffnung zum Einführen des Befestigungselements, beispielsweise einer Knochenschraube, versehen werden muss. Vielmehr kann das mindestens eine Fixierelement derart am Implantat angeordnet werden, dass es zum Beispiel einseitig und/oder unsymmetrisch in die Durchbrechung hineinragt oder vom Implantat vorsteht, um die freie Querschnittsfläche der Durchbrechung zu verringern. Dadurch wird zudem auch das Anordnen des Fixierelements, insbesondere das Festlegen desselben, am Implantat, deutlich vereinfacht, da beispielsweise das Fixierelement in eine dafür vorgesehene Aufnahme eingesetzt werden kann. Diese muss insbesondere nicht in Form einer Ringnut ausgebildet sein, sondern kann die Form einer einfachen Bohrung aufweisen. Ferner ermöglicht es die erfindungsgemäß vorgeschlagene Weiterbildung, das Implantat so klein auszubilden, dass es auch bei Frakturen im Handwurzelbereich, zum Beispiel bei einer Fraktur des Radius, zum Einsatz kommen kann. In einem solchen Fall kann das Implantat insbesondere intramedullär in den Radius eingebracht werden. Ferner eignet sich das Implantat auch zur extramedullären Verankerung an einem Knochen, beispielsweise an einem Humerus. Des Weiteren kann das Implantat auch so ausgebildet sein, dass das Fixierelement in mehr als eine Durchbrechung hineinragt. Beispielsweise kann auch mit einem Fixierelement die Bewegung von zwei, drei oder mehr Befestigungselementen und des Implantats relativ zueinander gebremst werden. Vorteilhaft ist es, dass das mindestens eine Fixierelement an der Durchbrechung und/oder in einer Fixierelementaufnahme unbeweglich festgelegt ist. Dadurch kann eine Baugröße des Implantats minimal gehalten werden.

Grundsätzlich wäre es denkbar, dass das mindestens eine Fixierelement selbst mit einer Durchbrechung versehen ist. Um jedoch die Herstellung des Implantats zu vereinfachen, ist es vorteilhaft, wenn das mindestens eine Fixierelement durchbrechungsfrei ausgebildet ist.

Um eine Relativbewegung zwischen einem Befestigungselement, beispielsweise einer Knochenschraube, und dem Implantat zu minimieren, wäre es vorteilhaft, wenn die Durchbrechung mit einem Gewinde oder mit Gewindeabschnitten versehen ist, die zu einem Außengewinde einer Knochenschraube korrespondieren, die die Durchbrechung zum Festlegen des Implantats an einem Knochenteil durchsetzt. Um jedoch eine Knochenschraube möglichst schnell, nämlich ohne sie in die Durchbrechung einschrauben zu müssen, in die Durchbrechung einführen zu können, ist es vorteilhaft, wenn die mindestens eine Durchbrechung vollständig gewindefrei ausgebildet ist.

Vorteilhafterweise ist das mindestens eine Fixierelement teilweise in eine mit der mindestens einen Durchbrechung verbundene Fixierelementaufnahme eingesetzt. Es wäre zwar durchaus denkbar, das Fixierelement auf das Implantat aufzusetzen, so dass eine freie Querschnittsfläche der Durchbrechung durch das Fixierelement verringert wird, ohne die Durchbrechung vollständig zu umgeben. Das Fixierelement in eine Fixierelementaufnahme einzusetzen hat jedoch den Vorteil, dass am Implantat keine unnötigen Vorsprünge ausgebildet werden, die zur Verbindung von Knochenteilen nicht erforderlich sind. So kann insgesamt eine Baugröße des Implantats minimiert werden.

Um das Implantat möglichst stabil auszubilden, ist es vorteilhaft, wenn die Fixierelementaufnahme in Form einer quer oder im Wesentlichen quer zur Durchbrechungsrichtung angeordneten Ausnehmung ausgebildet ist. Dies hat zudem den Vorzug, dass das in die Fixierelementaufnahme eingesetzte Fixierelement nicht in Folge einer auf es einwirkenden Kraft parallel zur Durchbrechungsrichtung aus der Fixierelementaufnahme herausbewegt werden kann. Je nach Ausgestaltung und Formgebung des Fixierelements kann es beispielsweise bei dieser Anordnung auch quer zur Knochenschraube teilweise in einen oder mehrere Gewindegänge einer Knochenschraube eintauchen.

Besonders einfach herzustellen ist die Ausnehmung, wenn diese einen kreisförmigen oder eckigen Querschnitt aufweist. Insbesondere kann ein korrespondierend geformtes Fixierelement formschlüssig in eine derartige Ausnehmung eingesetzt werden.

Damit das mindestens eine Fixierelement besonders einfach am Implantat angeordnet werden kann, ist es günstig, wenn die mindestens eine Fixierelementaufnahme mindestens eine Aufnahmerichtung definiert und wenn das mindestens eine Fixierelement parallel zur mindestens einen Aufnahmerichtung in die mindestens eine Fixierelementaufnahme eingesetzt und/oder teilweise einführbar ist.

Die Herstellung des Implantats vereinfacht sich weiter, wenn die Fixierelementaufnahme in Form einer eine erste Aufnahmebohrungslängsachse definierenden Aufnahmebohrung ausgebildet ist und wenn die Aufnahmebohrungslängsachse die Aufnahmerichtung definiert. Die Aufnahmebohrung kann das Implantat insbesondere vollständig durchsetzen oder nur teilweise, also auch in Form einer Sacklochbohrung ausgebildet sein. Eine Sacklochbohrung hat zudem den Vorteil, dass sich das mindestens eine Fixierelement an einem verschlossenen Ende derselben abstützen und nicht aus dieser austreten kann.

Gemäß einer bevorzugten Ausführungsform der Erfindung kann vorgesehen sein, dass die Durchbrechungsrichtung und die mindestens eine Aufnahmerichtung parallel zueinander verlaufen. Dies gestattet es, die Fixierelementaufnahme beispielsweise durch Vorsehen einer zur Durchbrechung seitlich etwas versetzten Bohrung auszubilden, wobei die Durchbrechung und die Fixierelementaufnahme nur durch Verschieben des Implantats hergestellt werden können, jedoch ohne das Implantat drehen zu müssen.

Besonders vorteilhaft ist es, wenn die Durchbrechungsrichtung und die mindestens eine Aufnahmerichtung windschief zueinander verlaufen. Dadurch wird insbesondere die Stabilität des Implantats erhöht, denn die Fixierelementaufnahme und die Durchbrechung können mit einer nur minimalen Überlappung ausgebildet werden.

Um eine möglichst große Überlappung zwischen der Fixierelementaufnahme und der Durchbrechung zu erreichen, ist es günstig, wenn die Durchbrechungsrichtung und die mindestens eine Aufnahmerichtung einander schneiden.

Die Herstellung des Implantats kann weiter vereinfacht werden, wenn die Durchbrechung in Form einer eine Bohrungslängsachse definierenden Bohrung ausgebildet ist und wenn die Bohrungslängsachse die Durchbrechungsrichtung definiert.

Um das mindestens eine Fixierelement am Implantat sicher festzulegen, ist es vorteilhaft, wenn das mindestens eine Fixierelement an der mindestens einen Fixierelementaufnahme kraftschlüssig und/oder mindestens teilweise formschlüssig gehalten ist. Beispielsweise kann es in der Fixierelementaufnahme klemmend gehalten sein.

Das mindestens eine Fixierelement und damit das Implantat sind besonders einfach herzustellen, wenn das mindestens eine Fixierelement in Form eines zylindrischen oder quaderförmigen Körpers ausgebildet ist. Beispielsweise können Fixierelemente aus zylindrischen, quaderförmigen oder im Querschnitt quadratischen Profilen hergestellt werden.

Um Abstoßungsreaktionen nach einer Implantation des Implantats in einem menschlichen oder tierischen Körper zu vermeiden, ist es vorteilhaft, wenn das mindestens eine Fixierelement aus einem körperverträglichen Material hergestellt ist.

Günstig ist es, wenn das mindestens eine Fixierelement aus einem Kunststoff hergestellt ist. Ein Kunststoff lässt sich besonders einfach verarbeiten, ist kostengünstig und leicht.

Damit das mindestens eine Fixierelement dauerhaft seinen Zweck erfüllen kann, eine Relativbewegung zwischen einem in die mindestens eine Durchbrechung eingesetzten Befestigungselement und dem Implantat zu verhindern oder zu minimieren, ist es günstig, wenn der Kunststoff ein nicht resorbierbarer Kunststoff ist.

Vorzugsweise ist der Kunststoff Polyetheretherketon (PEEK), Silikon, ein Polyetheretherketon (PEEK) enthaltender oder ein Silikon enthaltender Kunststoff. Diese Materialien eignen sich hervorragend als implantierbare Kunststoffe.

Damit sich das mindestens eine Fixierelement optimal an ein Befestigungselement anpassen kann, ist es vorteilhaft, wenn es aus einem plastisch und/oder elastisch verformbaren Material hergestellt ist. Dadurch ist es möglich, dass nach Einführen eines Befestigungselements das mindestens eine Fixierelement mindestens teilweise aus der Durchbrechung zurückgedrängt wird, wodurch es zu einer Klemmung zwischen dem mindestens einen Fixierelement, dem Befestigungselement und dem Implantat kommen kann. Insbesondere ermöglicht die erfindungsgemäße Anordnung des Fixierelements, dass ein Befestigungselement mindestens einseitig am Implantat anliegt und von dem mindestens einen Fixierelement klemmend gegen das Implantat gedrückt oder an diesem gehalten wird.

Dabei kann es besonders günstig sein, wenn das plastisch verformbare Material durch auf es einwirkende Außenflächen eines Befestigungselements, insbesondere Gewindeflanken eines Gewindes einer Schraube, plastisch und/oder elastisch verformbar ist. Auf diese Weise kann eine Relativbewegung zwischen dem Implantat und einem Befestigungselement minimiert oder sogar völlig ausgeschlossen werden.

Um eine Relativbewegung zwischen dem Implantat und einem Befestigungselement zu verhindern, ist es grundsätzlich nicht zwingend erforderlich, dass das mindestens eine Fixierelement starr mit dem Implantat verbunden ist. Vorteilhaft ist es jedoch, wenn das mindestens eine Fixierelement am Implantat unbeweglich angeordnet ist. Dadurch kann insbesondere vermieden werden, dass sich das mindestens eine Fixierelement vom Implantat löst, wenn kein Befestigungselement die mindestens eine Durchbrechung durchsetzt.

Eine besonders sichere Verbindung zwischen dem mindestens einen Fixierelement und dem Implantat lässt sich erreichen, wenn das mindestens eine Fixierelement in die Durchbrechung und/oder in die Fixierelementaufnahme eingepresst oder eingeschweißt ist. Denkbar wäre es auch, das mindestens eine Fixierelement durch Kleben mit dem Implantat zu verbinden.

Besonders einfach lässt sich das mindestens eine Fixierelement mit dem Implantat verbinden, wenn es in die Durchbrechung und/oder die Fixierelementaufnahme eingeschraubt ist.

Dabei kann es günstig sein, wenn die Durchbrechung und/oder die Fixierelementaufnahme mit einem Innengewinde versehen sind und wenn das mindestens eine Fixierelement mit einem zum Innengewinde korrespondierenden Außengewinde versehen ist. Dies ermöglicht es, das mindestens eine Fixierelement mit dem Implantat zu verschrauben.

Gemäß einer weiteren bevorzugten Ausführungsform der Erfindung kann ferner vorgesehen sein, dass das mindestens eine Fixierelement am Implantat mit einem Fixierelementbefestigungselement festgelegt ist. Beispielsweise kann das mindestens eine Fixierelement auch rastend mit dem Implantat verbunden werden, so dass das Fixierelementbefestigungselement durch ein Rastglied gebildet wird. Denkbar wäre auch die Festlegung des Fixierelements mittels einer ein Fixierelementbefestigungselement bildenden Madenschraube.

Vorzugsweise ist das mindestens eine Fixierelement am Implantat lösbar verbindbar angeordnet. Dies gestattet es, das mindestens eine Fixierelement vom Implantat zu lösen und auszuwechseln, beispielsweise wenn es dauerhaft verformt wurde oder wenn eine größere Verkleinerung einer freien Querschnittsfläche der Durchbrechung des Implantats gewünscht ist.

Um zwei Knochenteile auf einfache Weise miteinander verbinden zu können, ist es vorteilhaft, wenn das Implantat in Form einer Knochenplatte ausgebildet ist.

Gemäß einer weiteren bevorzugten Ausführungsform der Erfindung kann das Implantat auch in Form eines Knochennagels ausgebildet sein. Denkbar wäre es auch, das Implantat in Form eines Knochennagels auszubilden, welcher einstückig an eine Knochenplatte angeformt ist.

Um das Implantat an einem menschlichen oder tierischen Körper festlegen zu können, ist es vorteilhaft, wenn es mindestens ein Befestigungselement zum Festlegen an einen menschlichen oder tierischen Körper umfasst.

Besonders sicher lässt sich das erfindungsgemäße Implantat an einem menschlichen oder tierischen Körper festlegen, beispielsweise an Knochenteilen derselben, wenn das mindestens eine Befestigungselement in Form einer Knochenschraube ausgebildet ist.

Die nachfolgende Beschreibung einer bevorzugten Ausführungsform der Erfindung dient im Zusammenhang mit der Zeichnung der näheren Erläuterung. Es zeigen:
- Figur 1:: eine schematische perspektivische Darstellung eines an einem Kno- chen festgelegten erfindungsgemäßen Implantats;

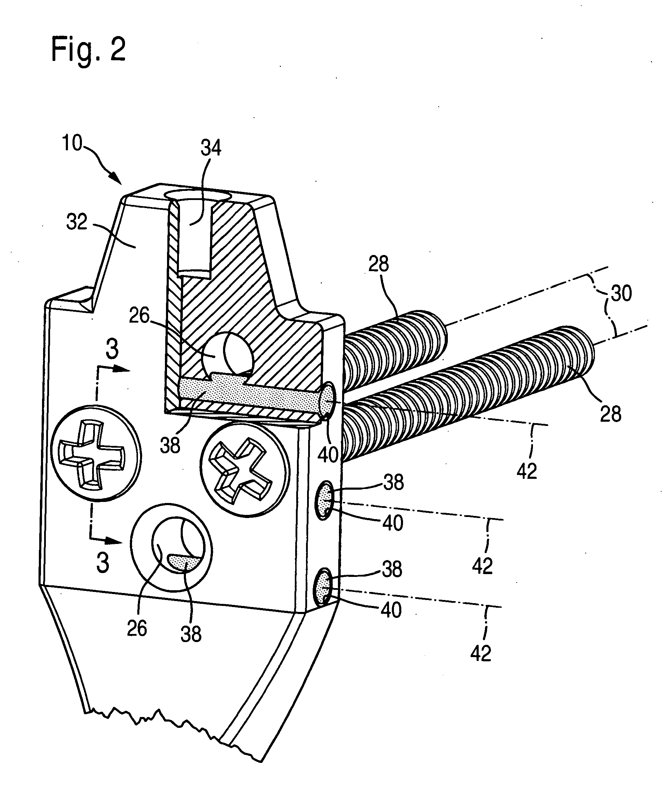

- Figur 2:: eine teilweise geschnittene perspektivische Ansicht eines Teils des Implantats aus Figur 1; und
- Figur 3:: eine Schnittansicht längs Linie 3-3 in Figur 2.

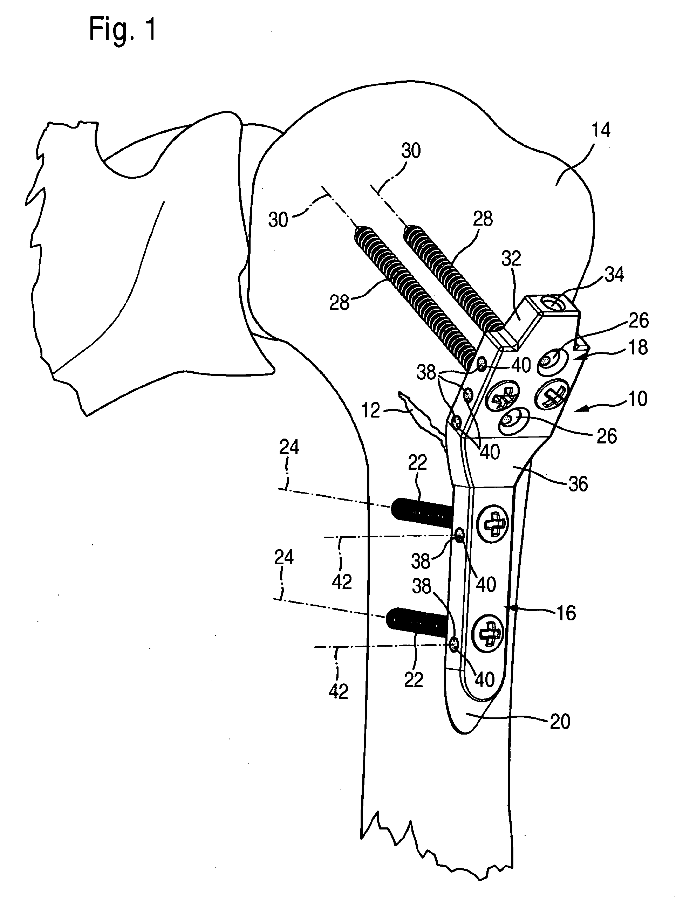

In Figur 1 ist ein insgesamt mit dem Bezugszeichen 10 versehenes Implantat in Form eines Verriegelungsnagels dargestellt, das zum Verbinden zweier Knochenteile oder, wie in Figur 1 zu sehen, zum Stabilisieren einer Teilfraktur 12 eines Knochens 14 eines menschlichen oder tierischen Körpers verwendet werden kann.

Das Implantat 10 umfasst im Wesentlichen zwei einstückig miteinander verbundene Teile, nämlich einen stabförmigen Abschnitt 16 und einen relativ zu diesem abgewinkelten plattenförmigen Abschnitt 18. Ein distales Ende 20 des stabförmigen Abschnitts 16 ist keilförmig geformt, um das Einführen des Implantats 10 in einen menschlichen oder tierischen Körper zu erleichtern und um Gewebe beim Einführen des Implantats 10 nicht zu verletzen. Der im Querschnitt quaderförmige Abschnitt 16, der und/oder dessen distales Ende 20 optional auch ganz oder abschnittsweise gekrümmt sein können, ist mit zwei in Figur 1 nicht erkennbaren, Durchbrechungen bildenden Bohrungen versehen, die zur Aufnahme von jeweils einer in Befestigungselement bildenden Knochenschraube 22 dienen, mit der der Abschnitt 16 am Knochen 14 festlegbar ist. Längsachsen 24 der Bohrungen fallen mit Längsachsen der Knochenschrauben 22 zusammen und definieren eine Durchbrechungsrichtung.

Der plattenförmige Abschnitt 18 ist mit insgesamt vier Durchbrechungen bildenden Bohrungen 26 versehen, die ebenfalls zur Aufnahme von Befestigungselementen, beispielsweise in Form von Knochenschrauben 28, dienen. Die Bohrungen 26, aber auch die Bohrungen am Abschnitt 16, können vollständig oder teilweise mit einem Innengewinde versehen sein, welches zu einem Außengewinde der Knochenschrauben 22 beziehungsweise 28 korrespondiert, so dass diese mit dem Implantat 10 verschraubt werden können. Längsachsen 30 der Knochenschrauben 28 fallen mit Längsachsen der Bohrungen 26 zusammen und definieren so eine Durchbrechungsrichtung der Durchbrechungen bildenden Bohrungen 26.

Ein proximaler Endabschnitt 32 des plattenförmigen Abschnitts 18 ist in Form eines trapezförmigen Vorsprungs ausgebildet, der sich in proximaler Richtung, also vom Abschnitt 16 weg weisend, etwas verjüngt. Der Endabschnitt 32 ist ferner mit einer Sacklochbohrung 34 versehen, die in proximaler Richtung weisend geöffnet ist. Der Endabschnitt 32 in Verbindung mit der Sacklochbohrung 34 bildet ein Kupplungselement, um das Implantat 10 beispielsweise mit einem korrespondierenden Kupplungselement eines Zielgeräts zum Setzen von Bohrungen im Knochen 14 zur Aufnahme der Knochenschrauben 22 und 28 lösbar zu verbinden.

Der im Vergleich zum Abschnitt 16 etwa doppelt so breite Abschnitt 18 verjüngt sich in einem Übergangsbereich 36 kontinuierlich bis zu einer Breite des Abschnitts 16. Der Übergangsbereich 36 ist zudem etwas gekrümmt, um die relativ zueinander abgewinkelten Abschnitte 16 und 18 knickfrei miteinander zu verbinden.

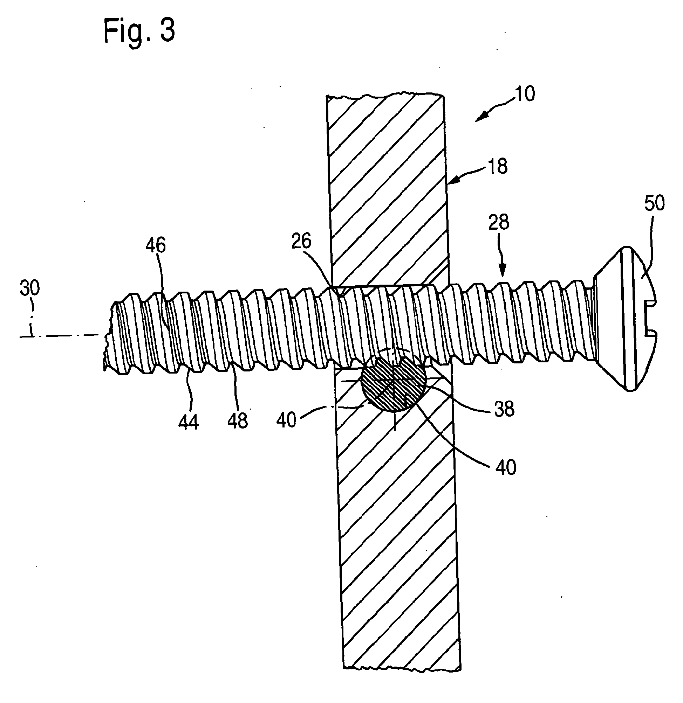

Da die Bohrungen 26 und auch die am Abschnitt 16 angeordneten Bohrungen bei dem in den Figuren dargestellten Ausführungsbeispiel nicht mit einem Innengewinde versehen sind, sind zur Verhinderung einer Relativbewegung zwischen dem Implantat 10 und den Knochenschrauben 22 und 28 insgesamt fünf stabförmige Fixierelemente 38 vorgesehen, die derart am Implantat 10 angeordnet sind, dass eine freie Querschnittsfläche der Durchbrechungen bildenden Bohrungen 26 und der am Abschnitt 16 vorgesehenen Bohrungen teilweise verkleinert wird, und zwar ohne dass die Fixierelemente die jeweilige Bohrung vollständig umgeben. Dies wird bei dem in den Figuren dargestellten Ausführungsbeispiel dadurch erreicht, dass für jedes der Fixierelemente 38 eine Fixierelementaufnahme 40 vorgesehen ist, und zwar in Form einer Bohrung, deren Längsachse 42 mit einer Längsachse des in Form eines zylindrischen Stabs ausgebildeten Fixierelements 38 zusammenfällt. Die Fixierelementaufnahmen 40 sind derart angeordnet, dass ihre Längsachsen 42 windschief zu den Längsachsen der zugeordneten Bohrungen 26 beziehungsweise der am Abschnitt 16 vorgesehenen Bohrungen verlaufen, sie also nicht schneiden. Die Längsachsen 24 und 28 sind jeweils senkrecht zu den Längsachsen 42 orientiert. Die Fixierelemente 38 können parallel zu den Längsachsen 42 in die Fixierelementaufnahmen 40 eingeschoben werden. Die Fixierelemente 38 ragen, da die Fixierelementaufnahmen 40 teilweise mit den Bohrungen 26 beziehungsweise den in Abschnitt 16 vorgesehenen Bohrungen überlappen, wie exemplarisch in Figur 3 zu erkennen ist, teilweise in die Durchbrechungen bildenden Bohrungen 26 beziehungsweise am Abschnitt 16 vorgesehenen Bohrungen hinein. So ist es möglich, dass die Fixierelemente 38 die Knochenschrauben 22 und 28 klemmend in den jeweiligen Bohrungen 26 beziehungsweise den im Abschnitt 16 vorgesehenen Bohrungen halten.

Die Fixierelemente 38 sind vorzugsweise aus einem körperverträglichen Material hergestellt, insbesondere aus einem Kunststoff. Der Kunststoff ist vorzugsweise nicht resorbierbar. Es handelt sich beim Kunststoff zum Beispiel um Polyetheretherketon (PEEK), Silikon, einen Polyetheretherketon (PEEK) enthaltenden oder einen Silikon enthaltenden Kunststoff. Insbesondere sind die Fixierelemente 38 aus einem Material hergestellt, das plastisch und/oder elastisch verformbar ist. Dies ermöglicht es, dass beispielsweise eine in die Bohrung 26 mit ihrem ein Außengewinde 44 aufweisenden Schaft 46 eingeschobene Knochenschraube 28 das teilweise vorstehende Fixierelement 38 teilweise zusammendrückt und plastisch und/oder elastisch verformt, so dass das Fixierelement 38, wie in Figur 3 dargestellt, mindestens teilweise in einen Gewindegang 48 des Außengewindes 44 eingreifen kann. Die Knochenschraube 28 wird so durch das Fixierelement 38 klemmend in der Bohrung 26 gehalten. Die Knochenschraube wird so also zwischen dem Implantat 10 und dem Fixierelement 38 klemmend gehalten. Je nach Materialwahl für das Fixierelement 38 kann die Knochenschraube 28 in der Bohrung 26 verschoben oder in diese weiter eingeschraubt werden, bis ein Kopf 50 der Knochenschraube 28 am Abschnitt 18 anschlägt.

An Stelle der Knochenschrauben 22 und 28 können optional auch Knochennägel verwendet werden, wobei die Fixierelemente 38 die Knochennägel kraftschlüssig in den jeweiligen Bohrungen halten. In jedem Fall wird ein Verkippen des Befestigungselements in der Durchbrechung des Implantats 10 minimiert und insbesondere bei den Knochenschrauben 22 und 28 ein Herauswandern derselben sowohl aus dem Knochen 14 als auch aus den jeweiligen Bohrungen des Implantats 10 verhindert.

Die Fixierelemente 38 können in die Fixierelementaufnahmen 40 form- oder kraftschlüssig eingesetzt sein. Insbesondere dann, wenn sich das Fixierelement 38 plastisch und/oder elastisch verformen lässt, kann es auf einfache Weise kraftschlüssig in die Fixierelementaufnahme eingesetzt werden. Alternativ ist es auch möglich, die Fixierelementaufnahme 40 mit einem Innengewinde zu versehen, mit welchem ein mit einem korrespondierenden Außengewinde versehenes Fixierelement 38 verschraubt werden kann. Eine weitere Alternative zur Festlegung der Fixierelemente 38 am Implantat 10 besteht darin, diese mittels eines Fixierelementbefestigungselements am Implantat 10 zu sichern, beispielsweise mit einer Madenschraube. Ferner kann das Fixierelement 38 in der Fixierelementaufnahme 40 oder am Implantat dadurch gesichert werden, dass es angeschweißt oder angeklebt wird.

In den Figuren nicht dargestellt ist eine weitere alternative Anordnung eines Fixierelements am Implantat 10, nämlich das Fixierelement auf einer äußeren Oberfläche der Abschnitte 16 und 18 benachbart den jeweiligen Bohrungen anzuordnen, so dass diese teilweise überdeckt werden.

Selbstverständlich können jeder Durchbrechung des Implantats 10 auch mehrere Fixierelemente 38 zugeordnet sein, die beispielsweise symmetrisch zur Durchbrechung oder unsymmetrisch zu derselben angeordnet sind.

Das Implantat 10 kann beispielsweise, wie oben beschrieben, extramedullär an einem Knochen 14 festgelegt werden. Abhängig von seiner Größe kann es jedoch auch intramedullär in einen Knochen eingebracht werden, beispielsweise bei einer Fraktur im Handwurzelbereich in den sogenannten Radius-Knochen. Wird das Implantat intramedullär in einen Knochen eingebracht, so werden die Köpfe 50 der verwendeten Knochenschrauben 22 und 28 nicht am Implantat 10 verankert, sondern am Knochen, in den das Implantat 10 eingebracht wurde.

Des Weiteren können Fixierelemente, wie beispielsweise in Figur 3 zu erkennen, auch derart angeordnet sein, dass ein einziges Fixierelement 38 eine freie Querschnittsfläche von zwei oder auch mehr Durchbrechungen teilweise verkleinert. Dies verringert insgesamt den Herstellungsaufwand, da nicht jeder Durchbrechung ein eigenes Fixierelement 38 zugeordnet werden muss.

## Patentansprüche

1. Implantat (10) mit mindestens einer eine Durchbrechungsrichtung (24, 30) definierenden Durchbrechung (26) zum Aufnehmen eines Befestigungselements (22, 28) zum Festlegen des Implantats (10) an oder in einem menschlichen oder tierischen Körper, wobei das Implantat (10) mindestens ein Fixierelement (38) umfasst, welches derart am Implantat (10) angeordnet ist und mindestens teilweise in die mindestens eine Durchbrechung (26) vorsteht, dass eine freie Querschnittsfläche der mindestens einen Durchbrechung (26) durch das mindestens eine Fixierelement (38) mindestens teilweise verkleinert wird, wobei das mindestens eine Fixierelement (38), ohne die mindestens eine Durchbrechung (26) vollständig zu umgeben, am Implantat (10) angeordnet ist, **dadurch gekennzeichnet, dass** das mindestens eine Fixierelement (38) an der Durchbrechung (26) und/oder in einer Fixierelementaufnahme (40) unbeweglich festgelegt ist.

2. Implantat nach Anspruch 1, **dadurch gekennzeichnet, dass** das mindestens eine Fixierelement (38) durchbrechungsfrei ausgebildet ist.

3. Implantat nach einem der voranstehenden Ansprüche, **dadurch gekennzeichnet, dass** das mindestens eine Fixierelement (38) teilweise in eine mit der mindestens einen Durchbrechung (26) verbundene Fixierelementaufnahme (40) eingesetzt ist.

4. Implantat nach Anspruch 3, **dadurch gekennzeichnet, dass** die mindestens eine Fixierelementaufnahme (40) mindestens eine Aufnahmerichtung (42) definiert und dass das mindestens eine Fixierelement (38) parallel zur mindestens einen Aufnahmerichtung (42) in die mindestens eine Fixierelementaufnahme (40) eingesetzt und/oder teilweise einführbar ist.

5. Implantat nach einem der voranstehenden Ansprüche, **dadurch gekennzeichnet, dass** die Durchbrechung (26) in Form einer eine Bohrungslängsachse (24, 30) definierenden Bohrung (26) ausgebildet ist und dass die Bohrungslängsachse (24, 30) die Durchbrechungsrichtung (24, 30) definiert.

6. Implantat nach Anspruch 5, **dadurch gekennzeichnet, dass** das mindestens eine Fixierelement (38) in der mindestens einen Fixierelementaufnahme (40) kraftschlüssig und/oder mindestens teilweise formschlüssig gehalten ist.

7. Implantat nach einem der voranstehenden Ansprüche, **dadurch gekennzeichnet, dass** das mindestens eine Fixierelement (38) aus einem Kunststoff hergestellt ist.

8. Implantat nach Anspruch 7, **dadurch gekennzeichnet, dass** der Kunststoff ein nichtresorbierbarer Kunststoff ist.

9. Implantat nach einem der Ansprüche 7 oder 8 , **dadurch gekennzeichnet, dass** der Kunststoff Polyetheretherketon (PEEK), Silikon, ein Polyetheretherketon (PEEK) enthaltender oder ein Silikon enthaltender Kunststoff ist.

10. Implantat nach einem der Ansprüche 8 oder 9, **dadurch gekennzeichnet, dass** die Durchbrechungsrichtung (24, 30) und die mindestens eine Aufnahmerichtung (42) windschief zueinander verlaufen.

11. Implantat nach einem der voranstehenden Ansprüche, **dadurch gekennzeichnet, dass** das mindestens eine Fixierelement (38) in die Durchbrechung (26) und/oder in die Fixierelementaufnahme (40) eingepresst oder eingeschweißt ist.

12. Implantat nach einem der voranstehenden Ansprüche, **dadurch gekennzeichnet, dass** das mindestens eine Fixierelement (38) in die Durchbrechung (26) und/oder die Fixierelementaufnahme (40) eingeschraubt ist.

13. Implantat nach Anspruch 12, **dadurch gekennzeichnet, dass** die Durchbrechung (26) und/oder die Fixierelementaufnahme (40) mit einem Innengewinde versehen sind und dass das mindestens eine Fixierelement (38) mit einem zum Innengewinde korrespondierenden Außengewinde versehen ist.

14. Implantat nach einem der voranstehenden Ansprüche, **dadurch gekennzeichnet, dass** das mindestens eine Fixierelement (38) am Implantat (10) mit einem Fixierelementbefestigungselement festgelegt ist.

15. Implantat nach einem der voranstehenden Ansprüche, **dadurch gekennzeichnet, dass** das Implantat (10) in Form eines Knochennagels ausgebildet ist.

## Claims

1. Implant (10) with at least one opening (26) defining an opening direction (24, 30) for receiving a fastening element (22, 28) for securing the implant (10) to or in a human or animal body, the implant (10) comprising at least one fixing element (38) which is arranged in such a way on the implant (10) and projects at least partially into the at least one opening (26) that a free cross-sectional area of the at least one opening (26) is at least partially reduced in size by the at least one fixing element (38), and the at least one fixing element (38) being arranged on the implant (10) without completely surrounding the at least one opening (26), **characterized in that** the at least one fixing element (38) is immovably secured at the opening (26) and/or in a fixing element receptacle (40).

2. Implant in accordance with claim 1, **characterized in that** the at least one fixing element (38) is of opening-free configuration.

3. Implant in accordance with any one of the preceding claims, **characterized in that** the at least one fixing element (38) is partially inserted into a fixing element receptacle (40) which is connected to the at least one opening (26).

4. Implant in accordance with claim 3, **characterized in that** the at least one fixing element receptacle (40) defines at least one receiving direction (42), and **in that** the at least one fixing element (38) is inserted and/or partially introducible into the at least one fixing element receptacle (40) parallel to the at least one receiving direction (42).

5. Implant in accordance with any one of the preceding claims, **characterized in that** the opening (26) is constructed in the form of a bore (26) defining a longitudinal bore axis (24, 30), and **in that** the longitudinal bore axis (24, 30) defines the opening direction (24, 30).

6. Implant in accordance with claim 5, **characterized in that** the at least one fixing element (38) is held in the at least one fixing element receptacle (40) with force locking and/or at least partially with positive locking.

7. Implant in accordance with any one of the preceding claims, **characterized in that** the at least one fixing element (38) is made of a plastic material.

8. Implant in accordance with claim 7, **characterized in that** the plastic material is a non-absorbable plastic material.

9. Implant in accordance with claim 7 or 8, **characterized in that** the plastic material is polyetheretherketone (PEEK), silicone, a plastic material containing polyetheretherketone (PEEK) or a plastic material containing silicone.

10. Implant in accordance with claim 8 or 9, **characterized in that** the opening direction (24, 30) and the at least one receiving direction (42) extend in a skew manner in relation to each other.

11. Implant in accordance with any one of the preceding claims, **characterized in that** the at least one fixing element (38) is pressed or welded into the opening (26) and/or into the fixing element receptacle (40).

12. Implant in accordance with any one of the preceding claims, **characterized in that** the at least one fixing element (38) is screwed into the opening (26) and/or the fixing element receptacle (40).

13. Implant in accordance with claim 12, **characterized in that** the opening (26) and/or the fixing element receptacle (40) are provided with an internal thread, and **in that** the at least one fixing element (38) is provided with an external thread corresponding to the internal thread.

14. Implant in accordance with any one of the preceding claims, **characterized in that** the at least one fixing element (38) is secured to the implant (10) with a fixing element fastening element.

15. Implant in accordance with any one of the preceding claims, **characterized in that** the implant (10) is constructed in the form of a bone nail.

## Revendications

1. Implant (10) comportant au moins un orifice de passage (26) qui définit une direction de passage (24, 30) et est destiné à recevoir un élément de fixation (22, 28) pour fixer l'implant (10) sur ou dans un corps humain ou animal, l'implant (10) comprenant au moins un élément d'arrêt (38) qui est agencé sur l'implant (10) et fait saillie dans ledit au moins un orifice de passage (26) de façon telle qu'une surface de section transversale dudit au moins un orifice de passage (26) soit au moins partiellement diminuée par ledit au moins un élément d'arrêt (38), ledit au moins un élément d'arrêt (38) étant agencé sur l'implant (10) sans entourer totalement ledit au moins un orifice de passage (26), **caractérisé en ce que** ledit au moins un élément d'arrêt (38) est fixé de manière non mobile au niveau de l'orifice de passage (26) et/ou dans un logement de réception d'élément d'arrêt (40).

2. Implant selon la revendication 1, **caractérisé en ce que** ledit au moins un élément d'arrêt (38) est réalisé exempt d'orifice de passage.

3. Implant selon l'une des revendications précédentes, **caractérisé en ce que** ledit au moins un élément d'arrêt (38) est inséré partiellement dans un logement de réception d'élément d'arrêt (40) relié audit au moins un orifice de passage (26).

4. Implant selon la revendication 3, **caractérisé en ce que** ledit au moins un logement de réception d'élément d'arrêt (40) définit au moins une direction de logement de réception (42), et **en ce que** ledit au moins un élément d'arrêt (38) est inséré et/ou peut être partiellement introduit dans ledit au moins un logement de réception d'élément d'arrêt (40), parallèlement à ladite au moins une direction de logement de réception (42).

5. Implant selon l'une des revendications précédentes, **caractérisé en ce que** l'orifice de passage (26) est réalisé sous la forme d'un alésage (26) définissant un axe longitudinal d' alésage (24, 30), et **en ce que** l' axe longitudinal d'alésage (24, 30) définit la direction de passage (24, 30).

6. Implant selon la revendication 5, **caractérisé en ce que** ledit au moins un élément d'arrêt (38) est maintenu dans ledit au moins un logement de réception d'élément d'arrêt (40) moyennant une liaison par adhérence et/ou au moins partiellement moyennant une liaison par complémentarité de formes.

7. Implant selon l'une des revendications précédentes, **caractérisé en ce que** ledit au moins un élément d'arrêt (38) est fabriqué en une matière plastique.

8. Implant selon la revendication 7, **caractérisé en ce que** la matière plastique est une matière plastique non résorbable.

9. Implant selon l'une des revendications 7 ou 8, **caractérisé en ce que** la matière plastique est constituée par un polyéther éther cétone (PEEK), des silicones, une matière plastique renfermant un polyéther éther cétone (PEEK) ou une matière plastique renfermant des silicones.

10. Implant selon l'une des revendications 8 ou 9, **caractérisé en ce que** la direction de passage (24, 30) et ladite au moins une direction de logement de réception (42) s'étendent de manière gauche l'une par rapport à l'autre.

11. Implant selon l'une des revendications précédentes, **caractérisé en ce que** ledit au moins un élément d'arrêt (38) est monté à force ou soudé dans l'orifice de passage (26) et/ou dans le logement de réception d' élément d'arrêt (40).

12. Implant selon l'une des revendications précédentes, **caractérisé en ce que** ledit au moins un élément d'arrêt (38) est vissé dans l'orifice de passage (26) et/ou dans le logement de réception d'élément d'arrêt (40).

13. Implant selon la revendication 12, **caractérisé en ce que** l'orifice de passage (26) et/ou le logement de réception d'élément d'arrêt (40) sont munis d'un filetage intérieur, et **en ce que** ledit au moins un élément d'arrêt (38) est pourvu d'un filetage extérieur correspondant au filetage intérieur.

14. Implant selon l'une des revendications précédentes, **caractérisé en ce que** ledit au moins un élément d'arrêt (38) est fixé et immobilisé sur l'implant (10) au moyen d'un élément de fixation d'élément d'arrêt.

15. Implant selon l'une des revendications précédentes, **caractérisé en ce que** l'implant (10) est réalisé sous la forme d'un clou ou d'une broche à os.
